# EUROPEAN PATENT SPECIFICATION

(11) **EP 2 710 983 B1**
(45) Date of publication and mention of the grant of the patent: **20.12.2017**
(21) Application number: 13185218.8
(22) Date of filing: 19.09.2013
(51) Int. Cl.: A61F 2/915

(54) **Polymeric stent**
Polymerstent
Endoprothèse polymère

(30) Priority: 20.09.2012 KR 20120104473
(43) Date of publication of application: 26.03.2014
(73) Proprietor: Dotter Intellectual Pte. Ltd., Singapore 079903 (SG)
(72) Inventor: Kim, Hyung Il, 153-704 Seoul (KR)
(74) Representative: Dörries, Hans Ulrich

(56) References cited:
- WO-A1-97/09945
- US-A1- 2004 215 312
- US-A1- 2010 244 334
- US-A1- 2010 274 349

## Description

### TECHNICAL FIELD

The present invention relates to a polymeric stent, and to an endoprosthesis that is implanted within blood vessels and is formed of a polymer.

### BACKGROUND ART

In general, stents are expandable medical prostheses, and are used within body vessels of humans for a variety of medical applications.

Examples thereof include intravascular stents for treating stenoses, and stents for maintaining openings in the urinary, biliary, tracheobronchial, esophageal, and renal tracts, and inferior vena cava.

Typically, a delivery device that retains a stent in the compressed state is used to deliver the stent to a treatment site through body vessels.

In percutaneous transluminal angioplasty, an implantable endoprosthesis, that is, a stent is introduced through a delivery device, and is passed through vessel conduits to a treatment site. After the stent approaches the treatment site, the stent is mechanically expanded, usually with the aid of an inflatable balloon, thereby being expanded within the body vessel. The delivery device is then retreated and removed from the patient. The stent remains within the vessel at the treatment site as an implant. An example of an expandable endovascular stent with a flexible tubular body with a longitudinal axis is described in WO 97/09945.

### SUMMARY OF THE INVENTION

The present invention relates to a polymeric stent, and has been made in an effort to provide an endoprosthesis that is implanted within blood vessels and is formed of a polymer.

An exemplary embodiment of the present invention provides a polymeric stent including:

a repeating unit including a unit cell having a shape of a closed figure that looks like a V-letter and including a first hinge portion bent inwardly to the unit cell and a second hinge portion facing the first hinge portion and bent outwardly from the unit cell; and a linker portion extended outwardly from a bent portion of the second hinge portion

in which the repeating units are disposed such that an end of a linker portion of one repeating unit is connected to a bent portion of a first hinge portion of another adjacent repeating unit, and

an end of a first hinge portion and an end of a second hinge portion of one repeating unit are connected to an end of a first hinge portion and an end of a second hinge portion of the another adjacent repeating unit, respectively.

Another exemplary embodiment of the present invention provides a method for manufacturing a polymeric stent, including: forming a repeating unit including a unit cell having a shape of a closed figure that looks like a V-letter and including a first hinge portion bent inwardly to the unit cell and a second hinge portion facing the first hinge portion and bent outwardly from the unit cell; and a linker portion extended outwardly from a bent portion of the second hinge portion; and

disposing the repeating units, such that an end of a linker portion of one repeating unit is connected to a bent portion of a first hinge portion of another adjacent repeating unit, and

forming the repeating units continuously such that an end of a first hinge portion and an end of a second hinge portion of one repeating unit are connected to an end of a first hinge portion and an end of a second hinge portion of another adjacent repeating unit, respectively.

The polymeric stent according to an exemplary embodiment of the present invention is advantageous in that the mechanical strength thereof is high.

The polymeric stent according to an exemplary embodiment of the present invention is easily handled when inserted into body vessels because the strut thereof is thin.

The polymeric stent according to an exemplary embodiment of the present invention is advantageous in that the form thereof is slightly distorted by resistance when the polymeric stent is implanted into blood vessels.

The polymeric stent according to an exemplary embodiment of the present invention is advantageous in that the patterns thereof are not entangled with each other when the polymeric stent is expanded.

The polymeric stent according to an exemplary embodiment of the present invention is advantageous in that the polymeric stent has good flexibility because the strut thereof is relatively thin.

### BRIEF DESCRIPTION OF THE DRAWINGS

FIG. 1 is a schematic view of a pattern of a polymeric stent according to an exemplary embodiment of the present invention.
FIG. 2 is an enlarged view of FIG. 1.
FIG. 3 illustrates the shape of a pattern of the polymeric stent according to an exemplary embodiment of the present invention in each state of (a) when the polymeric stent is crimped, or (b) when the polymeric stent is expanded, respectively.
FIG. 4 illustrates the change in shape of the unit cell according to the interior angle of the second hinge portion of the polymeric stent according to an exemplary embodiment of the present invention.
FIG. 5 illustrates the change in pattern according to the thickness of the strut of the polymeric stent according to an exemplary embodiment of the present invention.

### DETAILED DESCRIPTION

The polymeric stent of the present invention includes: a repeating unit including a unit cell having a shape of a closed figure that looks like a V-letter and including a first hinge portion bent inwardly to the unit cell and a second hinge portion facing the first hinge portion and bent outwardly from the unit cell;
a linker portion extended outwardly from a bent portion of the second hinge portion.

In an exemplary embodiment of the present invention, the repeating units may be disposed such that an end of a linker portion of one repeating unit is connected to a bent portion of a first hinge portion of another adjacent repeating unit, or
an end of a first hinge portion and an end of a second hinge portion of one repeating unit may be disposed so as to be connected to an end of a first hinge portion and an end of a second hinge portion of the adjacent other repeating unit, respectively.

The polymeric stent according to an exemplary embodiment of the present invention includes three or more repeating units,
the repeating units may be disposed such that an end of a linker portion of one repeating unit is connected to a bent portion of a first hinge portion of another adjacent repeating unit, and
an end of a first hinge portion and an end of a second hinge portion of one repeating unit may be disposed so as to be connected to an end of a first hinge portion and an end of a second hinge portion of another adjacent repeating unit, respectively.

The polymeric stent of the present invention may be manufactured by disposing one or more of the repeating unit in a row.

In an exemplary embodiment of the present invention, when the polymeric stent is a tube type, 4 to 15 repeating units may be disposed in a circumferential direction of the polymeric stent.

The polymeric stent according to an exemplary embodiment of the present invention may be formed in the form of a cylinder by connecting one end of the pattern of the polymeric stent manufactured by repeating the repeating unit of the present invention to the other end thereof.

In an exemplary embodiment of the present invention, when an end of a first hinge portion and an end of a second hinge portion of one repeating unit are disposed so as to be connected to an end of a first hinge portion and an end of a second hinge portion of another adjacent repeating unit, respectively, a line in which one or more unit cells are consecutively disposed side by side and a line in which one or more linker portions among the repeating units are disposed spaced apart from each other are formed.

At this time, the line of the unit cell is defined as the (A) row, and the line of the linker portion is defined as the (B) row.

In an exemplary embodiment of the present invention, when the repeating unit of the present invention is repeatedly disposed in one direction and in the other direction vertical to the one direction, a closed figure may be formed even at the (B) row which is the line of the linker portion.

In an exemplary embodiment of the present invention, when the repeating unit of the present invention is repeatedly disposed in one direction and in the other direction vertical to the one direction, a closed figure of a V-letter type shape, which is positioned in a direction opposite to a unit cell having a shape of a closed figure that looks like a V-letter at the (A) row, may be formed at the (B) row which is a line of the linker portion.

In an exemplary embodiment of the present invention, when one end (a) of the pattern of the polymeric stent is connected to the other end (b) thereof, open figures at both ends of the (B) row may be connected to each other to form a closed figure.

In an exemplary embodiment of the present invention, when one end (a) of the pattern of the polymeric stent is connected to the other end (b) thereof, open figures at both ends of the (B) row may be connected to each other to form a closed figure having a V-letter type shape, which is positioned in a direction opposite to the unit cell having a shape of a closed figure that looks like a V-letter at the (A) row thereof.

In the repeating unit of an exemplary embodiment of the present invention, the length of the linker portion may be shorter than the distance between the bent portion of the first hinge portion and the bent portion of the second hinge portion.

Accordingly, the unit cell having a shape of a closed figure that looks like a V-letter at the (A) row may be larger than a closed figure having a V-letter type shape at the (B) row, which is formed by the linker portion. In addition, in a longitudinal direction of the linker portion, the height of the unit cell having a shape of a closed figure that looks like a V-letter at the (A) row may be larger than the height of a closed figure having a V-letter type shape at the (B) row, which is formed by the linker portion.

In the repeating unit of an exemplary embodiment of the present invention, the length of the linker portion may be longer than the distance between the bent portion of the first hinge portion and the bent portion of the second hinge portion.

Accordingly, the unit cell having a shape of a closed figure that looks like a V-letter at the (A) row may be smaller than a closed figure having a V-letter type shape at the (B) row, which is formed by the linker portion. Furthermore, in a longitudinal direction of the linker portion, the height of the unit cell having a shape of a closed figure that looks like a V-letter at the (A) row may be smaller than the height of a closed figure having a V-letter type shape at the (B) row, which is formed by the linker portion.

In the polymeric stent according to an exemplary embodiment of the present invention, a corner and an end of one repeating unit are connected to a corner and an end of another unit cell. That is, in the polymeric stent according to an exemplary embodiment of the present invention, a repeating unit does not have a corner or end which is not connected to each other.

The polymeric stent of the present invention does not have a corner or end which is not connected to each other, and thus is advantageous in that the polymeric stent of the present invention has a relatively higher mechanical strength than that of a polymeric stent that is formed of a strut having the same thickness.

The corner or end which is not connected to each other in a polymeric stent fails to serve to support the polymeric stent, and thus the polymeric stent of the present invention, which does not have a corner or end which is not connected to each other, has a relatively high mechanical strength.

In the same thickness, the polymeric stent has a relatively lower mechanical strength than that of a metal stent, and thus a strut of the polymeric stent is manufactured with a thickness larger than that of a strut of the metal stent.

The corner or end which is not connected to each other in the polymeric stent fails to serve to support the polymeric stent, and thus a strut is manufactured thicker than usual in order to impart a mechanical strength with a predetermined value or more.

However, the polymeric stent of the present invention does not have a corner or end which is not connected to each other, and thus is advantageous in that a mechanical strength with a predetermined value or more may be imparted with a relatively thin strut.

The polymeric stent of the present invention may be manufactured with a relatively thin strut, and thus is advantageous in that the polymeric stent has good flexibility.

In the polymeric stent according to an exemplary embodiment of the present invention, the thinner the thickness of the strut is manufactured, the smaller the size of the repeating unit becomes, thereby increasing the number of repeating units per the same area. Through this, the mechanical strength of the polymeric stent may be maintained.

Further, the polymeric stent in the related art is disadvantageous in that after the polymeric stent is implanted into the blood vessels, the corner which is not connected to each other may hamper the blood stream within the blood vessels and forms hematoma and the like. However, the polymeric stent of the present invention does not have a corner or end which is not connected to each other, and thus the aforementioned problem may be alleviated.

In the present invention, the corner means a point that is positioned at an end and a bent portion of the first hinge portion, an end and a bent portion of the second hinge portion, and an end of the linker portion in one repeating unit.

In an exemplary embodiment of the present invention, the interior angle of the second hinge portion may be 100 degrees or more.

In an exemplary embodiment of the present invention, the interior angle of the second hinge portion may be 90 to 160 degrees.

In an exemplary embodiment of the present invention, in the repeating unit, the area of a rectangle that passes through both ends of the first hinge portion, both ends of the second hinge portion and an end of the linker portion may be 0.1 to 45 mm² and 0.13 to 44.38 mm², if necessary. At this time, the rectangle includes rectangles and squares.

In an exemplary embodiment of the present invention, the repeating unit may include a strut of the unit cell, which is a line that forms the closed figure having a V-letter type shape of the unit cell and a strut of the linker portion, which is a line that forms the linker portion.

In an exemplary embodiment of the present invention, the repeating unit includes a strut of the unit cell, which is a line that forms the closed figure having a V-letter type shape of the unit cell and a strut of the linker portion, which is a line that forms the linker portion, and

in the polymeric stent, the area of the strut of the unit cell and the strut of the linker portion in the repeating unit may be 5 to 46%, based on the area of the rectangle which passes through both ends of the first hinge portion, both ends of the second hinge portion, and an end of the linker portion.

If necessary, the area of the strut of the unit cell and the strut of the linker portion in the repeating unit may be 15 to 40% and 25 to 35%, based on the area of the rectangle which passes through both ends of the first hinge portion, both ends of the second hinge portion, and an end of the linker portion.

At this time, in the repeating unit, the area of the rectangle that passes through both ends of the first hinge portion, both ends of the second hinge portion and an end of the linker portion means an area of a region that one unit cell and a linker portion thereof occupy, and the area of the strut of one unit cell and the strut of the linker portion means an area that the line of each strut occupies.

In an exemplary embodiment of the present invention, the thickness of the strut of the unit cell and the strut of the linker portion may be 80 to 160 µm.

At this time, the thickness of the strut means a length thereof in a direction that is vertical to the longitudinal direction. That is, the thickness of the strut means the line width of the strut.

In an exemplary embodiment of the present invention, when the polymeric stent is a tube type, the diameter of the polymeric stent may be 2 to 8 mm.

In the present invention, when the polymeric stent is a tube type, in a cross-section which is a direction that is vertical to the axis, the diameter of the polymeric stent means a length of a line segment that connects two points on the cross-section so as to pass through the center of the cross-section.

At this time, the diameter of the polymeric stent means a diameter of a polymeric stent formed by connecting one end of the pattern of the polymeric stent with the other end thereof.

In an exemplary embodiment of the present invention, the polymeric stent may include a biodegradable polymer.

The biodegradable polymer is not particularly limited as long as the polymer may be degraded in vivo.

For example, the biodegradable polymer may be a synthetic biodegradable polymer or natural biodegradable polymer.

The synthetic biodegradable polymer may be one polymer selected from polyglycolide, polylactide, poly p-dioxanone, polycaprolactone, trimethylene carbonate, polyhydroxyalkanoates, polypropylene fumarate, polyortho esters, other polyester, polyanhydride, polyphosphazenes, polyalkylcyanoacrylate, poloxamers, and polyamino L-tyrosine, or a copolymer thereof or a mixture thereof.

In addition, the natural biodegradable polymer may be a material selected from modified polysaccharides, oxidized cellulose, gelatin, and collagen, or a mixture of one or more thereof.

The stent of the present invention is an expandable medical prosthetic device, is implanted within body vessels of humans for a variety of medical applications, and serves to maintain the form of the body vessel.

The stent of the present invention may be used as intravascular stents for treating stenoses, stents for maintaining openings in the urinary, biliary, tracheobronchial, esophageal, and renal tracts, and inferior vena cava, and the like.

The stent of the present invention is delivered to a treatment site through various body vessels by using a delivery device. After the stent is positioned at the treatment site, the delivery device is actuated to release the stent and the stent is mechanically expanded, usually with the aid of an inflatable balloon, within the body vessel. The delivery device is then detached from the stent, and the stent remains within the body vessel at the treatment site as an implant.

The polymeric stent according to an exemplary embodiment of the present invention is advantageous in that the form thereof is less distorted by resistance when the polymeric stent is implanted into blood vessels.

The polymeric stent according to an exemplary embodiment of the present invention is advantageous in that the patterns thereof are not entangled with each other when the polymeric stent is expanded.

When the polymeric stent of the present invention is manufactured of a biodegradable polymer, the stent remains within the body vessel at the treatment site, is naturally degraded after a certain time, and thus may disappear.

At this time, time for the polymeric stent of the present invention to be degraded may be about 2 years.

In the polymeric stent according to an exemplary embodiment of the present invention, the repeating unit may be formed by processing a polymeric tube using laser. Specifically, the repeating unit of the present invention may be formed on the side surface of the polymeric tube.

The present invention provides a method for manufacturing a polymeric stent, the method including: forming a repeating unit including a unit cell having a shape of a closed figure that looks like a V-letter and including a first hinge portion bent inwardly to the unit cell and a second hinge portion facing the first hinge portion and bent outwardly from the unit cell; and a linker portion extended outwardly from a bent portion of the second hinge portion; and
disposing the repeating units, such that an end of a linker portion of one repeating unit is connected to a bent portion of a first hinge portion of another adjacent repeating unit, or
forming the repeating units continuously such that an end of a first hinge portion and an end of a second hinge portion of one repeating unit are connected to an end of a first hinge portion and an end of a second hinge portion of another adjacent repeating unit, respectively.

In an exemplary embodiment of the present invention, the repeating unit may be formed by using laser.

In an exemplary embodiment of the present invention, the repeating unit may be formed on the side surface of the polymeric tube.

In the present invention, the tube means one having a long axial length.

Hereinafter, preferred embodiments of the present invention will be described in more detail with reference to the accompanying drawings.

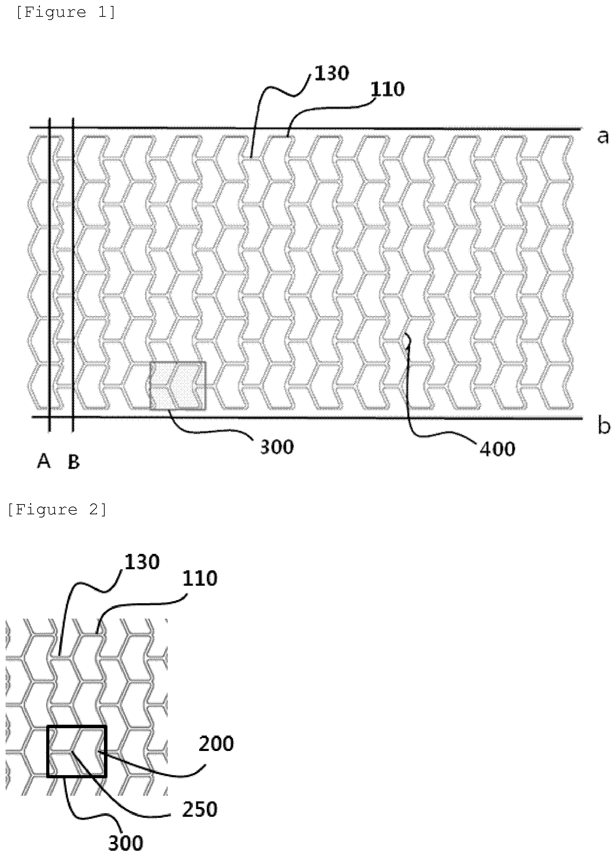

FIG. 1 of the present invention is a schematic view of the pattern of the polymeric stent according to an exemplary embodiment of the present invention, and FIG. 2 is an enlarged view of FIG. 1.

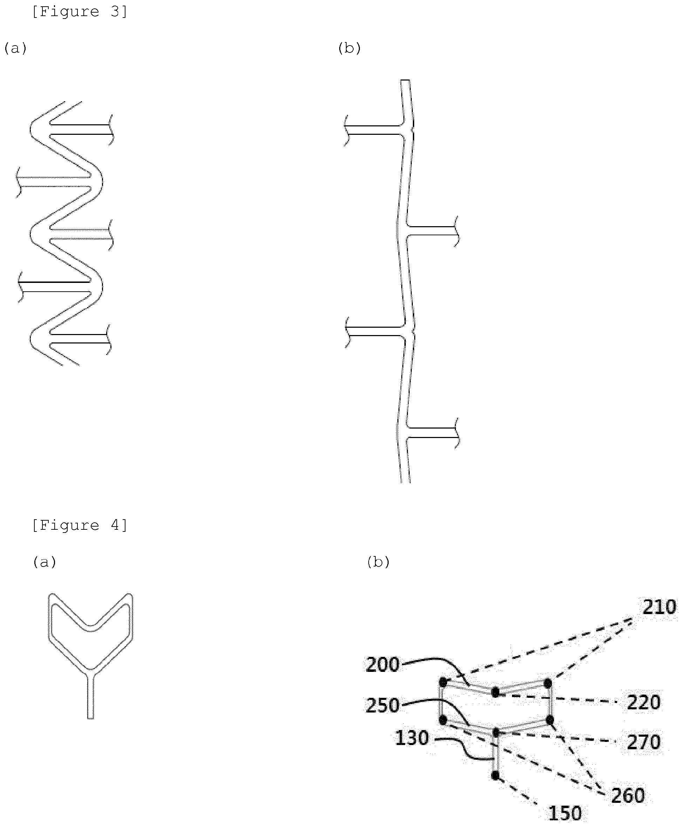

FIG. 3 of the present invention illustrates the shape of the pattern of the polymeric stent according to an exemplary embodiment of the present invention in each state of (a) when the polymeric stent is crimped, or (b) when the polymeric stent is expanded, respectively.

FIG. 4 of the present invention illustrates the change in shape of the unit cell according to the interior angle of the second hinge portion of the polymeric stent according to an exemplary embodiment of the present invention.

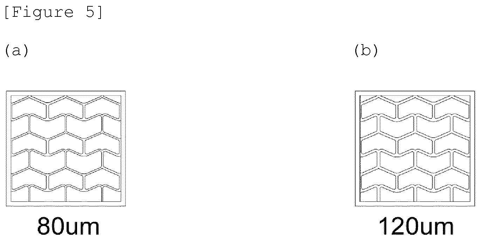

FIG. 5 of the present invention illustrates the change in pattern according to the thickness of the strut of the polymeric stent according to an exemplary embodiment of the present invention.

As illustrated in FIGS. 1 and 2, the polymeric stent of the present invention includes a unit cell 110 having a shape of a close figure that looks like a V-letter and including a first hinge portion 200 bent inwardly to the unit cell 110 and a second hinge portion 250 facing the first hinge portion 200 and bent outwardly from the unit cell; and
a repeating unit 300 including a linker portion 130 extended outwardly from a bent portion of the second hinge portion.

In an exemplary embodiment of the present invention, as illustrated in FIGS. 1 and 2, the repeating units 300 may be disposed such that an end of a linker portion of one repeating unit is connected to a bent portion of a first hinge portion of another adjacent repeating unit, or
an end of a first hinge portion and an end of a second hinge portion of one repeating unit are connected to an end of a first hinge portion and an end of a second hinge portion of another adjacent repeating unit, respectively.

The polymeric stent according to an exemplary embodiment of the present invention includes three or more repeating units,
the repeating units may be disposed such that an end of a linker portion of one repeating unit is connected to a bent portion of a first hinge portion of another adjacent repeating unit, and
an end of a first hinge portion and an end of a second hinge portion of one repeating unit are connected to an end of a first hinge portion and an end of a second hinge portion of further another adjacent repeating unit, respectively.

As illustrated in FIGS. 1 and 2, the polymeric stent of the present invention may be manufactured by disposing one or more of the repeating unit 300 in a row.

In an exemplary embodiment of the present invention, when the polymeric stent is a tube type, 4 to 15 repeating units may be disposed in a circumferential direction of the polymeric stent.

As illustrated in FIG. 1, the polymeric stent according to an exemplary embodiment of the present invention may be formed in the form of a cylinder by connecting one end (a) of the pattern of the polymeric stent manufactured by repeating the repeating unit 300 of the present invention to the other end (b) thereof. In an exemplary embodiment of the present invention, as illustrated in FIG. 1, a line disposed such that an end of a first hinge portion and an end of a second hinge portion of one unit cell are connected to an end of a first hinge portion and an end of a second hinge portion of another adjacent unit cell, respectively, is defined as the (A) row.

Furthermore, a line of the linker portion 130 formed according to the disposition of the unit cell is defined as the (B) row.

In an exemplary embodiment of the present invention, as illustrated in FIG. 1, when the repeating unit 300 of the present invention is repeatedly disposed in one direction and in the other direction vertical to the one direction, a closed figure may be formed even at the (B) row which is the line of the linker portion.

In an exemplary embodiment of the present invention, as illustrated in FIG. 1, when the repeating unit 300 of the present invention is repeatedly disposed in one direction and in the other direction vertical to the one direction, a closed figure having a V-letter type shape, which is positioned in a direction opposite to the unit cell having a shape of a closed figure that looks like a V-letter may be formed at the (B) row which is a line of the linker portion.

In an exemplary embodiment of the present invention, as illustrated in FIG. 1, when one end (a) of the pattern of the polymeric stent is connected to the other end (b) thereof, open figures at both ends of the (B) row may be connected to each other to form a closed figure.

In an exemplary embodiment of the present invention, as illustrated in FIG. 1, when one end (a) of the pattern of the polymeric stent is connected to the other end (b) thereof, open figures at both ends of the (B) row may be connected to each other to form a closed figure having a V-letter type shape, which is positioned in a direction opposite to the unit cell having a shape of a closed figure that looks like a V-letter at the (A) row thereof.

In the repeating unit 300 in an exemplary embodiment of the present invention, the length of the linker portion 130 may be shorter than the distance between the bent portion of the first hinge portion 200 and the bent portion of the second hinge portion 250.

Accordingly, in FIG. 1, the unit cell having a shape of a closed figure that looks like a V-letter at the (A) row may be larger than a closed figure having a V-letter type shape at the (B) row, which is formed by the linker portion. Further, in a longitudinal direction of the linker portion 130, the height of the unit cell having a shape of a closed figure that looks like a V-letter at the (A) row may be larger than the height of a closed figure having a V-letter type shape at the (B) row, which is formed by the linker portion.

In the repeating unit 300 in an exemplary embodiment of the present invention, the length of the linker portion 130 may be longer than the distance between the bent portion of the first hinge portion 200 and the bent portion of the second hinge portion 250.

Accordingly, in FIG. 1, the unit cell having a shape of a closed figure that looks like a V-letter at the (A) row may be smaller than a closed end having a V-letter type shape at the (B) row, which is formed by the linker portion. In addition, in a longitudinal direction of the linker portion 130, the height of the unit cell having a shape of a close figure that looks like a V-letter at the (A) row may be smaller than the height of a closed figure having a V-letter type shape at the (B) row, which is formed by the linker portion.

In the polymeric stent according to an exemplary embodiment of the present invention, as illustrated in FIGS. 1 and 2, a corner and an end of one repeating unit 300 are connected to a corner and an end of another unit cell. That is, in the polymeric stent according to an exemplary embodiment of the present invention, the repeating unit 300 does not have a corner or an end which is not connected to each other.

The polymeric stent of the present invention has no corner or end which is not connected to each other, and thus is advantageous in that the polymeric stent of the present invention has a relatively higher mechanical strength than that of a polymeric stent that is formed of a strut having the same thickness.

The corner or end which is not connected to each other in the polymeric stent fails to serve to support the polymeric stent, and thus the polymeric stent of the present invention, which does not have a corner or end which is not connected to each other, has a relatively high mechanical strength.

In the same thickness, the polymeric stent has a lower mechanical strength than that of a metal stent, and thus a strut of the polymeric stent is manufactured with a thickness larger than that of a strut of the metal stent.

The corner or end which is not connected to each other in the polymeric stent fails to serve to support the polymeric stent, and thus a strut is manufactured thicker than usual in order to impart a mechanical strength with a predetermined value or more.

However, the polymeric stent of the present invention does not have a corner or end which is not connected to each other, and thus is advantageous in that a mechanical strength with a predetermined value or more may be imparted with a relatively thin strut.

The polymeric stent of the present invention may be manufactured with a relatively thin strut, and thus is advantageous in that the polymeric stent has good flexibility.

In the polymeric stent according to an exemplary embodiment of the present invention, the thinner the thickness of the strut is manufactured, the smaller the size of the repeating unit becomes, thereby increasing the number of repeating units per the same area. Through this, the mechanical strength of the polymeric stent may be maintained.

Further, the polymeric stent in the related art is disadvantageous in that after the polymeric stent is implanted into blood vessels, the corner which is not connected to each other may hamper the blood stream within the blood vessels and forms hematoma and the like. However, the polymeric stent of the present invention does not have a corner or end which is not connected to each other, and thus the aforementioned problem may be alleviated.

In the present invention, the corner means a point that is positioned at an end and a bent portion of the first hinge portion, an end and a bent portion of the second hinge portion, and an end of the linker portion in one repeating unit.

In an exemplary embodiment of the present invention, the interior angle 400 of the second hinge portion may be 100 degrees or more.

In an exemplary embodiment of the present invention, the interior angle 400 of the second hinge portion may be 90 to 160 degrees or more.

FIG. 4 illustrates (a) a form of the unit cell when the interior angle 400 of the second hinge portion is 90 degrees and (b) a form of the unit cell when the interior angle 400 of the second hinge portion is 160 degrees.

In an exemplary embodiment of the present invention, in the repeating unit, the area of a rectangle that passes through both ends of the first hinge portion, both ends of the second hinge portion and an end of the linker portion may be 0.1 to 45 mm² and 0.13 to 44.38 mm², if necessary. At this time, the area of the rectangle means an area of a region that one unit cell and a linker portion thereof occupy. That is, the area of the rectangle means an area of the rectangle which is a region that one unit cell and a linker portion thereof, which are illustrated in FIGS. 1 and 2, occupy.

In an exemplary embodiment of the present invention, the repeating unit 300 includes a strut of the unit cell, which is a line that forms the closed figure that looks like a V-letter of the unit cell and a strut of the linker portion, which is a line that forms the linker portion, and

in the polymeric stent, the area of the strut of the unit cell and the strut of the linker portion in the repeating unit may be 5 to 46%, based on the area of the rectangle which passes through both ends of the first hinge portion, both ends of the second hinge portion, and an end of the linker portion.

If necessary, the area of the strut of the unit cell and the strut of the linker portion in the repeating unit may be 15 to 40% and 25 to 35%, based on the area of the rectangle which passes through both ends of the first hinge portion, both ends of the second hinge portion, and an end of the linker portion.

At this time, the area of the rectangle means an area of a region that one unit cell 110 and a linker portion 130 thereof occupy, and the area of the strut of one unit cell and the strut of the linker portion means an area that the line of each strut occupies.

In an exemplary embodiment of the present invention, the thickness of the strut of the unit cell and the strut of the linker portion may be 80 to 160 µm.

At this time, the thickness of the strut means a length thereof in a direction that is vertical to the longitudinal direction. That is, the thickness of the strut means the line width of the strut.

FIG. 5 illustrates (a) the pattern of the polymeric stent when the thickness of the unit cell 110 and the linker portion 130 is 80 µm and (b) the pattern of the polymeric stent when the thickness of the unit cell 110 and the linker portion 130 is 120 µm.

In an exemplary embodiment of the present invention, when the polymeric stent is a tube type, the diameter of the polymeric stent may be 2 to 8 mm.

In the present invention, when the polymeric stent is a tube type, in a cross-section in a direction that is vertical to the axis, the diameter of the polymeric stent means a length of a line segment that connects two points on the cross-section so as to pass through the center of the cross-section.

At this time, the diameter of the polymeric stent means a diameter of a polymeric stent formed by connecting an end a on one side of the pattern of the polymeric stent with an end b on the other side thereof in FIG. 1.

In an exemplary embodiment of the present invention, the polymeric stent may include a biodegradable polymer.
The biodegradable polymer is not particularly limited as long as the polymer may be degraded in vivo.

For example, the biodegradable polymer may be a synthetic biodegradable polymer or natural biodegradable polymer.

The synthetic biodegradable polymer may be one polymer selected from polyglycolide, polylactide, poly p-dioxanone, polycaprolactone, trimethylene carbonate, polyhydroxyalkanoates, polypropylene fumarate, polyortho esters, other polyester, polyanhydride, polyphosphazenes, polyalkylcyanoacrylate, poloxamers, and polyamino L-tyrosine, or a copolymer thereof or a mixture thereof.

In addition, the natural biodegradable polymer may be a material selected from modified polysaccharides, oxidized cellulose, gelatin, and collagen, or a mixture of one or more thereof.

The stent of the present invention is an expandable medical prosthetic device, is implanted within the body vessels of humans for a variety of medical applications, and serves to maintain the form of the body vessel.

The stent of the present invention may be used as intravascular stents for treating stenoses, stents for maintaining openings in the urinary, biliary, tracheobronchial, esophageal, and renal tracts, and inferior vena cava, and the like.

The stent of the present invention is delivered to a treatment site through various body vessels by using a delivery device. After the stent is positioned at the treatment site, the delivery device is actuated to release the stent and the stent is mechanically expanded, usually with the aid of an inflatable balloon, within the body vessel. The delivery device is then detached from the stent, and the stent remains within the body vessel at the treatment site as an implant.

The polymeric stent according to an exemplary embodiment of the present invention is advantageous in that the form thereof is slightly distorted by resistance when the polymeric stent is implanted into blood vessels.

The polymeric stent according to an exemplary embodiment of the present invention is advantageous in that the patterns thereof are not entangled with each other when the polymeric stent is expanded.

When the polymeric stent of the present invention is manufactured of a biodegradable polymer, the stent remains within the body vessel at the treatment site, is naturally degraded after a certain time, and thus may disappear.

At this time, time for the polymeric stent of the present invention to be degraded may be about 2 years.

In the polymeric stent according to an exemplary embodiment of the present invention, the repeating unit may be formed by processing a polymeric tube using laser. Specifically, the repeating unit of the present invention may be formed on the side surface of the polymeric tube.

Hereinafter, the present specification will be described in detail with respect to Examples. The following Examples are for illustrating the present invention, and the scope of the present invention includes the scope described in the following claims and the substitutions and modifications thereof, and is not limited to the scope of Examples.

In the polymeric stent of the present invention, when the length of the diameter, the number of patterns in a circumference and the thickness of the strut are modified, the interior angle of the second hinge portion, the area of the repeating unit, and the variations of the area ratio of the strut based on the area of the repeating unit are shown in the following Table 1.

**[Table 1]**

| Diameter length (mm) | | | 2mm | 3mm | 5mm | 6mm | 8mm |
|---|---|---|---|---|---|---|---|
| | Number of cells | Thickness (um) | | | | | |
| Area of minimum unit cell (mm²) | 4 ea | 80 | 2.57 | 6.31 | 17.9 | 25.05 | 44.38 |
| | | 120 | 2.43 | 6.12 | 17.6 | 24.86 | 43.88 |
| | | 160 | 2.32 | 6.11 | 17.58 | 24.67 | 43.38 |
| | 9 ea | 80 | 0.45 | 1.15 | 3.39 | 4.74 | 8.52 |
| | | 120 | 0.39 | 1.06 | 3.22 | 4.77 | 8.29 |
| | | 160 | 0.36 | 1.07 | 3.32 | 4.74 | 8.07 |
| | 15 ea | 80 | 0.13 | 0.35 | 1.11 | 1.59 | 3.21 |
| | | 120 | - | 0.30 | 1.01 | 1.49 | 2.79 |
| | | 160 | - | - | 0.92 | 1.39 | 2.66 |
| Area ratio (%) | 4 ea | 80 | 17 | 10 | 6 | 5 | 4 |
| | | 120 | 26 | 16 | 10 | 8 | 6 |
| | | 160 | 35 | 21 | 12 | 12 | 8 |
| | 9 ea | 80 | 38 | 24 | 14 | 13 | 9 |
| | | 120 | 58 | 37 | 22 | 18 | 14 |
| | | 160 | 74 | 46 | 27 | 24 | 19 |
| | 15 ea | 80 | 65 | 43 | 25 | 21 | 13 |
| | | 120 | - | 64 | 38 | 32 | 24 |
| | | 160 | - | - | 52 | 43 | 32 |
| V strut length (mm) | 4 ea | 80 | 1. 69 | 2.59 | 4.37 | 5.23 | 6.40 |
| | | 120 | 1.72 | 2.63 | 4.38 | 5.19 | 6.46 |
| | | 160 | 1.76 | 2.60 | 4.38 | 5.18 | 6.80 |
| | 9 ea | 80 | 0.79 | 1.18 | 1. 94 | 2.32 | 2.94 |
| | | 120 | 0.85 | 1.16 | 1. 95 | 2.31 | 2.84 |
| | | 160 | 0.91 | 1.16 | 1. 92 | 2.30 | 3.01 |
| | 15 ea | 80 | 0.48 | 0.72 | 1015 | 1.3 | 1.74 |
| | | 120 | - | 0.71 | 1.19 | 1.34 | 1.83 |
| | | 160 | - | - | 1.05 | 1.42 | 1.86 |

In the polymeric stent of the present invention, when the length of the diameter, the number of patterns in a circumference and the thickness of the strut are modified, the variations in the form of the unit cell are shown in the following Table 2.

**[Table 2]**

| Diameter length (mm) | | 2mm | 3mm | 5mm | 6mm | 8mm |
|---|---|---|---|---|---|---|
| Number of cells | Thickness (um) | | | | | |
| 4 ea | 80 | 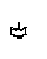 | 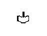 | 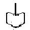 | 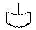 | 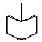 |
| | 120 | | 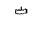 | 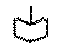 | 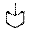 | 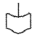 |
| | 160 | | | 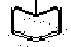 | 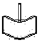 | 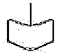 |
| 9 ea | 80 | | | 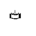 | 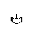 | 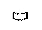 |
| | 120 | 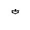 | | 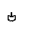 | 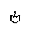 | 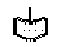 |
| | 160 | 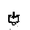 | 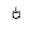 | 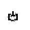 | 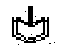 | 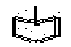 |
| 15 ea | 80 | | | 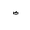 | 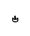 | 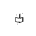 |
| | 120 | 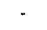 | | 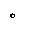 | 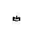 | 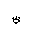 |
| | 160 | 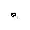 | 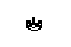 | 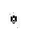 | 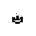 | 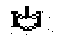 |

## Claims

1. A polymeric stent, comprising:
a repeating unit (200) comprising a unit cell (110) having a shape of a closed figure that looks like a V-letter and comprising a first hinge portion (200) bent inwardly to the unit cell and a second hinge portion (250) facing the first hinge portion and bent outwardly from the unit cell; and a linker portion (130) extended outwardly from a bent portion (270) of the second hinge portion,
wherein the polymeric stent comprises three or more repeating units,
the repeating units are disposed such that an end of a linker portion of one repeating unit is connected to a bent portion of a first hinge portion of another adjacent repeating unit, and an end of a first hinge portion and an end of a second hinge portion of one repeating unit are connected to an end of a first hinge portion and an end of a second hinge portion of further another adjacent repeating unit, respectively;
wherein the repeating units are manufactured from a biodegradable polymer selected from the group consisting of:
(a) a polyglycolide, a polylactide, a poly p-dioxanone, a polycaprolactone, a trimethylene carbonate, a polyhydroxyalkanoate, a polypropylene fumarate, a polyortho ester, a polyanhydride, a polyphosphazene, a polyalkylcyanoacrylate, a poloxamer, and a polyamino L-tyrosine, or a copolymer thereof or a mixture thereof; or
(b) a modified polysaccharide, an oxidized cellulose, a gelatin, and a collagen, or a mixture of one or more thereof;
wherein the thickness of the strut of the unit cell and the strut of the linker portion is 80 to 160 µm; and
wherein the patterns of the stent are not entangled with each other when the polymeric stent is expanded.

2. The polymeric stent of claim 1, wherein in the polymeric stent, all corners and ends of one repeating unit are connected to corners and ends of another adjacent repeating unit.

3. The polymeric stent of claim 1, wherein when the polymeric stent is a tube type, 4 to 15 repeating units are disposed in a circumferential direction of the polymeric stent.

4. The polymeric stent of claim 1, wherein an interior angle of the second hinge portion is 100 degrees or more.

5. The polymeric stent of claim 1, wherein in the repeating unit, an area of a rectangle that passes through both ends of the first hinge portion, both ends of the second hinge portion and an end of the linker portion is 0.1 to 45 mm².

6. The polymeric stent of claim 1, wherein the repeating unit comprises a strut of the unit cell, which is a line that forms the shape of a closed figure that looks like a V-letter of the unit cell and a strut of the linker portion, which is a line that forms the linker portion.

7. The polymeric stent of claim 6, wherein an area of the strut of the unit cell and the strut of the linker portion is 5 to 46%, based on an area of a rectangle which passes through both ends of the first hinge portion, both ends of the second hinge portion, and an end of the linker portion.

8. The polymeric stent of claim 1, wherein when the polymeric stent is a tube type, a diameter of the polymeric stent is 2 to 8 mm.

9. The polymeric stent of claim 1, wherein the repeating unit is formed by processing a polymeric tube using a laser.

10. The polymeric stent of claim 1, wherein the thickness of the strut of the unit cell and the strut of the linker portion is 80 to 120 µm.

11. A method for manufacturing the polymeric stent of claim 1 comprising:
forming a repeating unit (300) including a unit cell (100) having a shape of a closed figure that looks like a V-letter and including a first hinge portion (200) bent inwardly to the unit cell and a second hinge portion (250) facing the first hinge portion and bent outwardly from the unit cell; and a linker portion (130) extended outwardly from the bent portion (270) of the second hinge portion; and
disposing the repeating units, such that an end of a linker portion of one repeating unit is connected to a bent portion of a first hinge portion of another adjacent repeating unit, and forming the repeating units continuously such that an end of a first hinge portion and an end of a second hinge portion of one repeating unit are connected to an end of a first hinge portion and an end of a second hinge portion of the another adjacent repeating unit, respectively;
wherein the polymeric stent comprises three or more repeating units,
the repeating units are disposed such that an end of a linker portion of one repeating unit is connected to a bent portion of a first hinge portion of another adjacent repeating unit, and an end of a first hinge portion and an end of a second hinge portion of one repeating unit are connected to an end of a first hinge portion and an end of a second hinge portion of further another adjacent repeating unit, respectively;
wherein the repeating units are manufactured from a biodegradable polymer selected from the group consisting of:
(a) a polyglycolide, a polylactide, a poly p-dioxanone, a polycaprolactone, a trimethylene carbonate, a polyhydroxyalkanoate, a polypropylene fumarate, a polyortho ester, a polyanhydride, a polyphosphazene, a polyalkylcyanoacrylate, a poloxamer, and a polyamino L-tyrosine, or a copolymer thereof or a mixture thereof; or
(b) a modified polysaccharide, an oxidized cellulose, a gelatin, and a collagen, or a mixture of one or more thereof;
wherein the thickness of the strut of the unit cell and the strut of the linker portion is 80 to 160 µm; and
wherein the patterns of the stent are not entangled with each other when the polymeric stent is expanded.

12. The method of claim 11, wherein the repeating unit is formed by using a laser.

13. The method of claim 11 or 12, wherein the repeating unit is formed on a side surface of a polymeric tube.

14. The method of claim 11, wherein the thickness of the strut of the unit cell and the strut of the linker portion is 80 to 120 µm.

## Patentansprüche

1. Polymerer Stent, umfassend:
eine wiederholende Einheit (200), die eine Einheitszelle (110) umfasst, welche die Form einer geschlossenen Figur aufweist, die wie ein V-Buchstabe aussieht und einen ersten Gelenkabschnitt (200) umfasst, der nach innen zur Einheitszelle gebogen ist, und einen zweiten Gelenkabschnitt (250) umfasst, der dem ersten Gelenkabschnitt gegenüberliegt und von der Einheitsszelle nach außen gebogen ist; und einen Linkerabschnitt (130), der sich von einem gebogenen Abschnitt (270) des zweiten Gelenkabschnitts nach außen erstreckt,
wobei der polymere Stent drei oder mehr wiederholende Einheiten umfasst,
und die wiederholenden Einheiten so angeordnet sind, dass ein Ende eines Linkerabschnitts einer wiederholenden Einheit mit einem gebogenen Abschnitt eines ersten Gelenkabschnitts einer anderen anliegenden wiederholenden Einheit verbunden ist und ein Ende eines ersten Gelenkabschnitts und ein Ende eines zweiten Gelenkabschnitts einer wiederholenden Einheit jeweils mit einem Ende eines ersten Gelenkabschnitts und einem Ende eines zweiten Gelenkabschnitts von einer weiteren anderen anliegenden wiederholenden Einheit verbunden sind;
wobei die wiederholenden Einheiten aus biologisch abbaubarem Polymer hergestellt sind, das aus der Gruppe ausgewählt ist, bestehend aus:
(a) einem Polyglycolid, einem Polylactid, einem Poly-p-dioxanon, einem Polycaprolacton, einem Trimethylencarbonat, einem Polyhydroxyalkanoat, einem Polypropylenfumarat, einem Polyorthoester, einem Polyanhydrid, einem Polyphosphazen, einem Polyalkylcyanoacrylat, einem Poloxamer und einem Polyamino-L-tyrosin oder einem Copolymer davon oder einer Mischung davon; oder
(b) einem modifizierten Polysaccharid, einer oxidierten Zellulose, einem Gallert und einem Collagen oder einer Mischung von einem oder mehreren davon;
wobei die Dicke des Struts der Einheitszellen und des Struts des Linkerabschnitts 80 bis 160 µm beträgt, und
wobei die Muster des Stents sich nicht ineinander verfangen, wenn der polymere Stent aufgeweitet wird.

2. Polymerer Stent nach Anspruch 1, wobei im polymeren Stent alle Ecken und Enden einer wiederholenden Einheit mit den Ecken und Enden einer anderen anliegenden wiederholenden Einheit verbunden sind.

3. Polymerer Stent nach Anspruch 1, wobei der polymere Stent ein Röhrchentyp ist, und 4 bis 15 wiederholende Einheiten in einer Umfangsrichtung des polymeren Stents angeordnet sind.

4. Polymerer Stent nach Anspruch 1, wobei ein Innenwinkel des zweiten Gelenkabschnitts 100 Grad oder mehr beträgt.

5. Polymerer Stent nach Anspruch 1, wobei in der wiederholenden Einheit die Fläche eines Rechteckes, die durch beide Enden des ersten Gelenkabschnitts, beide Enden des zweiten Gelenkabschnitts und ein Ende des Linkerabschnitts hindurchgeht, 0,1 bis 45 mm² beträgt.

6. Polymerer Stent nach Anspruch 1, wobei die wiederholende Einheit einen Strut der Einheitszelle umfasst, eine Linie , welche die Form einer geschlossenen Figur bildet, die wie ein V-Buchstabe der Einheitszelle ausssieht, und einen Strut des Linkerabschnitts umfasst, eine Linie, die den Linkerabschnitt bildet.

7. Polymerer Stent nach Anspruch 6, wobei eine Fläche des Struts der Einheitszellen und des Struts des Linkerabschnitts 5 bis 46 % beträgt, basierend auf einer Fläche eines Rechtecks, die durch beide Enden des ersten Gelenksabschnitts, beide Enden des zweiten Gelenkabschnitts und ein Ende des Linkerabschnitts hindurchgeht.

8. Polymerer Stent nach Anspruch 1, wobei der polymere Stent ein Röhrchentyp ist und der Durchmesser des polymeren Stents 2 bis 8 mm beträgt.

9. Polymerer Stent nach Anspruch 1, wobei die wiederholende Einheit durch Bearbeiten eines polymeren Röhrchens mithilfe eines Lasers gebildet wird.

10. Polymerer Stent nach Anspruch 1, wobei die Dicke des Struts der Einheitszellen und des Struts des Linkerabschnitts 80 bis 120 µm beträgt.

11. Ein Verfahren zur Herstellung des polymeren Stents nach Anspruch 1, umfassend:
Ausbilden einer wiederholenden Einheit (300), die eine Einheitszelle (100) aufweist, welche die Form einer geschlossenen Figur zeigt, die wie ein V-Buchstabe aussieht und einen ersten Gelenkabschnitt (200) aufweist, der nach innen zur Einheitszelle gebogen ist, und einen zweiten Gelenkabschnitt (250) aufweist, welcher dem ersten Gelenkabschnitt gegenüberliegt und von der Einheitszelle nach außen gebogen ist; und einen Linkerabschnitt (130) aufweist, der sich vom gebogenen Abschnitt (270) des zweiten Gelenkabschnitts nach außen erstreckt; und
Anordnen der wiederholenden Einheiten derart, dass ein Ende eines Linkerabschnitts einer wiederholenden Einheit mit einem gebogenen Abschnitt eines ersten Gelenkabschnitts einer anderen anliegenden wiederholenden Einheit verbunden ist, und kontinuierliches Ausbilden der wiederholenden Einheiten derart, dass ein Ende eines ersten Gelenkabschnitts und ein Ende eines zweiten Gelenkabschnitts einer wiederholenden Einheit jeweils mit einem Ende eines ersten Gelenkabschnitts und einem Ende eines zweiten Gelenkabschnitts der weiteren anliegenden wiederholenden Einheit verbunden sind;
wobei der polymere Stent drei oder mehr wiederholende Einheiten umfasst,
und die wiederholenden Einheiten so angeordnet sind, dass ein Ende eines Linkerabschnitts einer wiederholenden Einheit mit einem gebogenen Abschnitt eines ersten Gelenkabschnitts einer anderen anliegenden wiederholenden Einheit verbunden ist und ein Ende eines ersten Gelenkabschnitts und ein Ende eines zweiten Gelenkabschnitts einer wiederholenden Einheit jeweils mit einem Ende eines ersten Gelenkabschnitts und einem Ende eines zweiten Gelenkabschnitts einer weiteren anderen anliegenden wiederholenden Einheit verbunden sind;
wobei die wiederholenden Einheiten aus einem biologisch abbaubaren Polymer hergestellt sind, das aus der Gruppe ausgewählt ist, bestehend aus:
(a) einem Polyglycolid, einem Polylactid, einem Poly-p-dioxanon, einem Polycaprolacton, einem Trimethylencarbonat, einem Polyhydroxyalkanoat, einem Polypropylenfumarat, einem Polyorthoester, einem Polyanhydrid, einem Polyphosphazen, einem Polyalkylcyanoacrylat, einem Poloxamer und einem Polyamino-L-tyrosin oder einem Copolymer davon oder einer Mischung davon; oder
(b) einem modifizierten Polysaccharid, einer oxidierten Zellulose, einem Gallert und einem Collagen oder einer Mischung von einem oder mehreren davon;
wobei die Dicke des Struts der Einheitszellen und des Struts des Linkerabschnitts 80 bis 160 µm beträgt, und
wobei die Muster des Stents sich nicht ineinander verfangen, wenn der polymere Stent aufgeweitet wird.

12. Verfahren nach Anspruch 11, wobei die wiederholende Einheit mithilfe eines Lasers ausgebildet wird.

13. Verfahren nach Anspruch 11 oder 12, wobei die wiederholende Einheit auf einer Seitenfläche eines polymeren Röhrchens ausgebildet wird.

14. Verfahren nach Anspruch 11, wobei die Dicke des Struts der Einheitszellen und des Struts des Linkerabschnitts 80 bis 120 µm beträgt.

## Revendications

1. Stent polymère, comprenant :
une unité répétée (200) comprenant une cellule unitaire (110) ayant une forme de figure fermée qui ressemble à la lettre V et comprenant une première partie charnière (200) courbée vers l'intérieur vers la cellule unitaire et une seconde partie charnière (250) tournée face à la première partie charnière et courbée vers l'extérieur de la cellule unitaire ; et une partie de liaison (130) qui s'étend vers l'extérieur à partir d'une partie courbée (270) de la seconde partie charnière,
dans lequel le stent polymère comprend trois unités répétées ou plus,
les unités répétées sont disposées de manière à ce qu'une extrémité d'une partie de liaison d'une unité répétée soit raccordée à une partie courbée de la première partie charnière d'une autre unité répétée adjacente, et une extrémité d'une première partie charnière et une extrémité d'une seconde partie charnière d'une unité répétée soient raccordées à une extrémité d'une première partie charnière et une extrémité d'une seconde partie charnière d'encore une autre unité, respectivement ;
dans lequel les unités répétées sont fabriquées à partir d'un polymère biodégradable sélectionné dans le groupe constitué de :
(a) un polyglycolide, un polylactide, un poly p-dioxanone, un polycaprolactone, un carbonate de triméthylène, un polyhydroxyalcanoate, un fumarate de polypropylène, un polyortho ester, un polyanhydride, un polyphosphazène, un polyalkylcyanoacrylate, un poloxamère, et une polyamino L-tyrosine, ou un copolymère de ceux-ci ou un mélange de ceux-ci ; ou
(b) un polysaccharide modifié, une cellulose oxydée, une gélatine, et un collagène, ou un mélange d'un ou plusieurs de ceux-ci ;
dans lequel l'épaisseur de l'entretoise de la cellule unitaire et de l'entretoise de la partie de liaison est de 80 à 160 µm ; et
dans lequel les motifs du stent ne s'enchevêtrent pas les uns les autres lorsque le stent polymère est déployé.

2. Stent polymère selon la revendication 1, dans lequel dans le stent polymère, tous les coins et toutes les extrémités d'une unité répétée sont raccordés aux coins et aux extrémités d'une autre unité répétée adjacente.

3. Stent polymère selon la revendication 1, dans lequel lorsque le stent polymère est un type de tube, 4 à 15 unités répétées sont disposées dans une direction circonférentielle du stent polymère.

4. Stent polymère selon la revendication 1, dans lequel un angle intérieur de la seconde partie charnière est de 100 degrés ou plus.

5. Stent polymère selon la revendication 1, dans lequel dans l'unité répétée, une aire d'un rectangle qui passe par les deux extrémités de la première partie charnière, les deux extrémités de la seconde partie charnière et une extrémité de la partie de liaison est de 0,1 à 45 mm².

6. Stent polymère selon la revendication 1, dans lequel l'unité répétée comprend une entretoise de la cellule unitaire, qui est une ligne qui prend la forme d'une figure fermée qui ressemble à la lettre V de la cellule unitaire et une entretoise de la partie de liaison, qui est une ligne qui forme la partie de liaison.

7. Stent polymère selon la revendication 6, dans lequel une aire de l'entretoise de la cellule unitaire et de l'entretoise de la partie de liaison est de 5 à 46 %, sur la base d'une aire d'un rectangle qui passe par les deux extrémités de la première partie charnière, les deux extrémités de la seconde partie charnière, et une extrémité de la partie de liaison.

8. Stent polymère selon la revendication 1, dans lequel lorsque le stent polymère est un type de tube, un diamètre du stent polymère est de 2 à 8 mm.

9. Stent polymère selon la revendication 1, dans lequel l'unité répétée est formée par traitement d'un tube polymère en utilisant un laser.

10. Stent polymère selon la revendication 1, dans lequel l'épaisseur de l'entretoise de la cellule unitaire et de l'entretoise de la partie de liaison est de 80 à 120 µm.

11. Procédé de fabrication du stent polymère selon la revendication 1, comprenant :
la formation d'une unité répétée (300) comprenant une cellule unitaire (100) ayant une forme de figure fermée qui ressemble à la lettre V et comprenant une première partie charnière (200) courbée vers l'intérieur vers la cellule unitaire et une seconde partie charnière (250) tournée face à la première partie charnière et courbée vers l'extérieur de la cellule unitaire; et une partie de liaison (130) qui s'étend vers l'extérieur à partir de la partie courbée (270) de la seconde partie charnière ; et
la disposition des unités répétées, de telle sorte qu'une extrémité d'une partie de liaison d'une unité répétée soit raccordée à une partie courbée d'une première partie charnière d'une autre unité répétée adjacente, et la formation des unités répétées de façon continue de telle sorte qu'une extrémité d'une première partie charnière et une extrémité d'une seconde partie charnière d'une unité répétée soient raccordées à une extrémité d'une première partie et à une extrémité d'une seconde partie charnière de l'autre unité répétée adjacente, respectivement ;
dans lequel le stent polymère comprend trois unités répétées ou plus,
les unités répétées sont disposées de manière à ce qu'une extrémité d'une partie de liaison d'une unité répétée soit raccordée à une partie courbée d'une première partie charnière d'une autre unité répétée adjacente, et une extrémité d'une première partie charnière et une extrémité d'une seconde partie charnière d'une unité répétée soient raccordées à une extrémité d'une première partie charnière et une extrémité d'une seconde partie charnière d'encore une autre unité répétée adjacente, respectivement ;
dans lequel les unités répétées sont fabriquées à partir d'un polymère biodégradable sélectionné dans le groupe constitué de :
(a) un polyglycolide, un polylactide, un poly p-dioxanone, un polycaprolactone, un carbonate de triméthylène, un polyhydroxyalcanoate, un fumarate de polypropylène, un polyortho ester, un polyanhydride, un polyphosphazène, un polyalkylcyanoacrylate, un poloxamère, et une polyamino L-tyrosine, ou un copolymère de ceux-ci ou un mélange de ceux-ci ; ou
(b) un polysaccharide modifié, une cellulose oxydée, une gélatine, et un collagène, ou un mélange d'un ou plusieurs de ceux-ci ;
dans lequel l'épaisseur de l'entretoise de la cellule unitaire et de l'entretoise de la partie de liaison est de 80 à 160 µm ; et
dans lequel les motifs du stent ne s'enchevêtrent pas les uns les autres lorsque le stent polymère est déployé.

12. Procédé selon la revendication 11, dans lequel l'unité répétée est formée en utilisant un laser.

13. Procédé selon la revendication 11 ou 12, dans lequel l'unité répétée est formée sur une surface latérale d'un tube polymère.

14. Procédé selon la revendication 11, dans lequel l'épaisseur de l'entretoise de la cellule unitaire et de l'entretoise de la partie de liaison est de 80 à 120 µm.
